# EUROPEAN PATENT APPLICATION

(11) **EP 4 686 458 A1**
(43) Date of publication of application: **04.02.2026**
(21) Application number: 25192102.9
(22) Date of filing: 28.07.2025
(51) Int. Cl.: A61F 2/40

(54) **GLENOID IMPLANTS WITH HYBRID FIXATION**

(30) Priority: 30.07.2024 US 202463676938 P
(71) Applicant: Howmedica Osteonics Corp., Mahwah, NJ 07430 (US)
(72) Inventor: Wolfe, Alexander Paul, Fort Wayne, 46804 (US); Stump, David R., Columbia City, 46725 (US); Nelson, Michael C., Syracuse, 46567 (US); Crous, Heinrich George, Killeagh, P36F291 (IE)
(74) Representative: Zimmermann & Partner Patentanwälte mbB

(57) **Abstract**

Disclosed are embodiments of anatomic glenoid implants that incorporate hybrid fixation.

## Description

### Cross-Reference to Related Applications

This application claims benefit under 35 U.S.C. § 119(e) to U.S. Provisional Application Serial No. 63/676,938, filed July 30, 2024, entitled "GLENOID IMPLANTS WITH HYBRID FIXATION," the disclosure of which is incorporated by reference herein in its entirety.

### Field of Disclosure

This disclosure relates generally to shoulder arthroplasty and more particularly to glenoid implant components.

### Background

Improving the stability of glenoid implants in shoulder arthroplasty is an ever-present challenge in shoulder implant design.

### Summary

Disclosed are embodiments of anatomic glenoid implants that incorporate hybrid fixation.

### Brief Description of the Drawings

The figures provided with this disclosure are schematic and are not necessarily to scale. The figures are not intended to show the actual dimensions or actual relative dimensions unless otherwise specified.
FIG. 1A illustrates an example of an anatomic glenoid implant having a hybrid fixation post, in accordance with embodiments of the present disclosure.
FIG. 1B illustrates a longitudinal section view of the hybrid fixation post portion of the anatomic glenoid implant of FIG. 1A, in accordance with some embodiments of the present disclosure.
FIG. 2A illustrates an example of the metallic portion of the hybrid fixation post, in accordance with some embodiments of the present disclosure.
FIG. 2B illustrates an example of the interdigitating lattice cone portion of the metallic portion of the hybrid fixation post in which the interdigitating lattice cone has a concave configuration, in accordance with some embodiments of the present disclosure.
FIG. 2C illustrates the structural details of the interdigitating lattice cone of FIG. 2B, in accordance with some embodiments of the present disclosure.
FIG. 2D illustrates an example of the interdigitating lattice portion of the metallic portion of the hybrid fixation post in which the interdigitating lattice has a flat configuration, in accordance with some embodiments of the present disclosure.
FIG. 3A illustrates a perspective view of another embodiment of a metallic portion of the hybrid fixation post, in accordance with some embodiments of the present disclosure.
FIG. 3B illustrates a side view of the metallic portion of the hybrid fixation post shown in FIG. 3A, in accordance with some embodiments of the present disclosure.
FIG. 3C illustrates a longitudinal section view of the metallic portion of FIGS. 3A and 3B, in accordance with some embodiments of the present disclosure.
FIG. 3D illustrates a longitudinal section view of a hybrid fixation post utilizing the embodiment of the metallic portion shown in FIGS. 3A-3C, in accordance with some embodiments of the present disclosure.
FIGS. 4A-4C illustrate an anatomic glenoid implant having a hybrid fixation post, in accordance with some embodiments of the present disclosure.
FIGS. 5A-5F illustrate augmented anatomic glenoid implants having a hybrid fixation post, in accordance with some embodiments of the present disclosure.
FIG. 6 illustrates a general process flow for fabricating a glenoid implant including a hybrid fixation post, in accordance with some embodiments of the present disclosure.
FIG. 7A illustrates a perspective view of another embodiment of a metallic distal portion of a hybrid fixation post including a conical porous metal structure having a first thickness, in accordance with some embodiments of the present disclosure.
FIG. 7B illustrates a side view of the metallic portion of the hybrid fixation post shown in FIG. 7A, in accordance with some embodiments of the present disclosure.
FIG. 7C illustrates a longitudinal section view of the metallic portion of FIGS. 7A and 7B, in accordance with some embodiments of the present disclosure.
FIG. 7D illustrates a cross-sectional view of the metallic portion of FIGS. 7A-7C, in accordance with some embodiments.
FIG. 8A illustrates a perspective view of another embodiment of a metallic distal portion of a hybrid fixation post including a conical porous metal structure having a second thickness, in accordance with some embodiments of the present disclosure.
FIG. 8B illustrates a side view of the metallic portion of the hybrid fixation post shown in FIG. 8A, in accordance with some embodiments of the present disclosure.
FIG. 8C illustrates a longitudinal section view of the metallic portion of FIGS. 8A and 8B, in accordance with some embodiments of the present disclosure.
FIG. 8D illustrates a cross-sectional view of the metallic portion of FIGS. 8A-8C, in accordance with some embodiments.
FIG. 9A illustrates a perspective view of another embodiment of a metallic distal portion of a hybrid fixation post including a porous metal structure defining a cavity, in accordance with some embodiments of the present disclosure.
FIG. 9B illustrates a side view of the metallic portion of the hybrid fixation post shown in FIG. 9A, in accordance with some embodiments of the present disclosure.
FIG. 9C illustrates a longitudinal section view of the metallic portion of FIGS. 9A and 9B, in accordance with some embodiments of the present disclosure.
FIG. 9D illustrates a cross-sectional view of the metallic portion of FIGS. 9A-9C, in accordance with some embodiments.
FIG. 10A illustrates a perspective view of another embodiment of a metallic distal portion of a hybrid fixation post including a groove, in accordance with some embodiments of the present disclosure.
FIG. 10B illustrates a side view of the metallic portion of the hybrid fixation post shown in FIG. 10A, in accordance with some embodiments of the present disclosure.
FIG. 10C illustrates a longitudinal section view of the metallic portion of FIGS. 10A and 10B, in accordance with some embodiments of the present disclosure.
FIG. 10D illustrates a cross-sectional view of the metallic portion of FIGS. 10A-10C, in accordance with some embodiments.
FIG. 11A illustrates a perspective view of another embodiment of a metallic distal portion of a hybrid fixation post including a macro-cross and porous metal structure, in accordance with some embodiments of the present disclosure.
FIG. 11B illustrates a side view of the metallic portion of the hybrid fixation post shown in FIG. 11A, in accordance with some embodiments of the present disclosure.
FIG. 11C illustrates a longitudinal section view of the metallic portion of FIGS. 11A and 11B, in accordance with some embodiments of the present disclosure.
FIG. 11D illustrates a cross-sectional view of the metallic portion of FIGS. 11A-11C, in accordance with some embodiments.
FIG. 12A illustrates a perspective view of another embodiment of a metallic distal portion of a hybrid fixation post including a radial mesh, in accordance with some embodiments of the present disclosure.
FIG. 12B illustrates a side view of the metallic portion of the hybrid fixation post shown in FIG. 12A, in accordance with some embodiments of the present disclosure.
FIG. 12C illustrates a longitudinal section view of the metallic portion of FIGS. 12A and 12B, in accordance with some embodiments of the present disclosure.
FIG. 12D illustrates a cross-sectional view of the metallic portion of FIGS. 12A-12C, in accordance with some embodiments.
FIG. 13A illustrates a perspective view of another embodiment of a metallic distal portion of a hybrid fixation post including a coarse mesh, in accordance with some embodiments of the present disclosure.
FIG. 13B illustrates a side view of the metallic portion of the hybrid fixation post shown in FIG. 13A, in accordance with some embodiments of the present disclosure.
FIG. 13C illustrates a longitudinal section view of the metallic portion of FIGS. 13A and 13B, in accordance with some embodiments of the present disclosure.
FIG. 13D illustrates a cross-sectional view of the metallic portion of FIGS. 13A-13C, in accordance with some embodiments.
FIG. 14A illustrates a perspective view of another embodiment of a metallic distal portion of a hybrid fixation post including a transformer, in accordance with some embodiments of the present disclosure.
FIG. 14B illustrates a side view of the metallic portion of the hybrid fixation post shown in FIG. 14A, in accordance with some embodiments of the present disclosure.
FIG. 14C illustrates a longitudinal section view of the metallic portion of FIGS. 14A and 14B, in accordance with some embodiments of the present disclosure.
FIG. 14D illustrates a cross-sectional view of the metallic portion of FIGS. 14A-14C, in accordance with some embodiments.

### Detailed Description

This description of the exemplary embodiments is intended to be read in connection with the accompanying drawings, which are to be considered part of the entire written description. The drawing figures are not necessarily to scale and certain features may be shown exaggerated in scale or in somewhat schematic form in the interest of clarity and conciseness. In the description, relative terms such as "horizontal," "vertical," "up", "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. When only a single machine is illustrated, the term "machine" shall also be taken to include any collection of machines that individually or jointly execute a set (or multiple sets) of instructions to perform any one or more of the methodologies discussed herein. The term "operatively connected" is such an attachment, coupling or connection that allows the pertinent structures to operate as intended by virtue of that relationship. In the claims, means-plus-function clauses, if used, are intended to cover the structures described, suggested, or rendered obvious by the written description or drawings for performing the recited function, including not only structural equivalents but also equivalent structures.

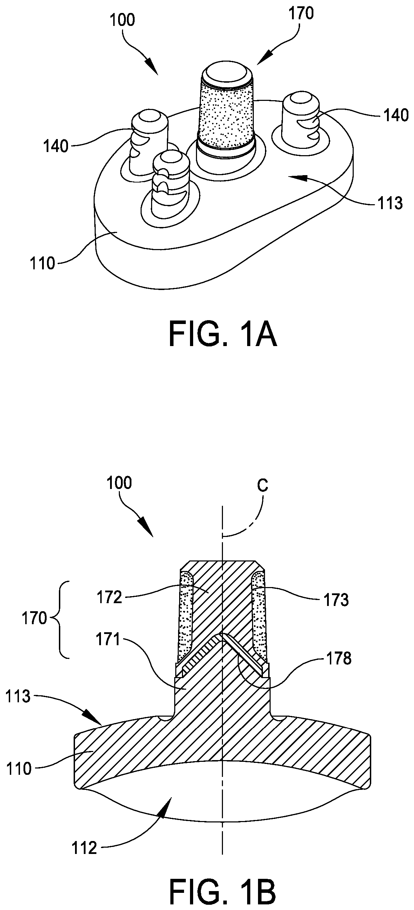

Referring to FIGS. 1A-2C, an improved anatomic glenoid implant 100 for implantation in a glenoid according to the present disclosure is provided. The glenoid implant 100 comprises an articular body 110, and at least one hybrid fixation post 170. The articular body 110 comprises an articulation surface 112 and a medial surface 113 on the opposite side of the articular body. The medial surface 113 is referred to herein as the "medial" surface because that is the surface that engages the prepared glenoid surface when implanted in a patient so when implanted, that surface faces in the medial direction in the patient's body. The term hybrid for hybrid fixation post refers to the hybrid composition of the post described further below.

The illustrated example of the articular body 110 has a circular or generally cylindrical shape when configured for inlaid applications where the glenoid implant is inset into a prepared glenoid. However, the articular body 110 can be formed to have different shapes to accommodate different conditions of the receiving glenoid. For those embodiments, the procedure for preparing the glenoid surface would be modified accordingly.

The hybrid fixation post 170 extends from the medial surface 113. The hybrid fixation post 170 comprises a polymer base portion 171 that is located proximally with respect to the articular body 110) and a metallic distal portion 172 that is located distally with respect to the articular body 110.

The hybrid fixation post 170 has a hybrid composition that is comprised of the base portion 171 and the distal portion 172. The base portion 171 is an extension of the articular body 110 which is generally made of a durable biocompatible polymer such as ultra-high-molecular-weight polyethylene (UHMWPE). The distal portion 172 comprises a metallic structure that is joined to the base portion 171.

In some embodiments, the distal portion 172 comprises a solid metallic core whose exterior side wall surface comprises a porous metallic layer 173 that promotes bone in-growth after implanted into a glenoid. The glenoid implant 100 can be provided in a variety of versions with varying dimensions and varying thickness of the porous metallic layer 173 to accommodate different anatomical conditions of patients' glenoid. The porous metallic layer 173 can be titanium or titanium based alloy.

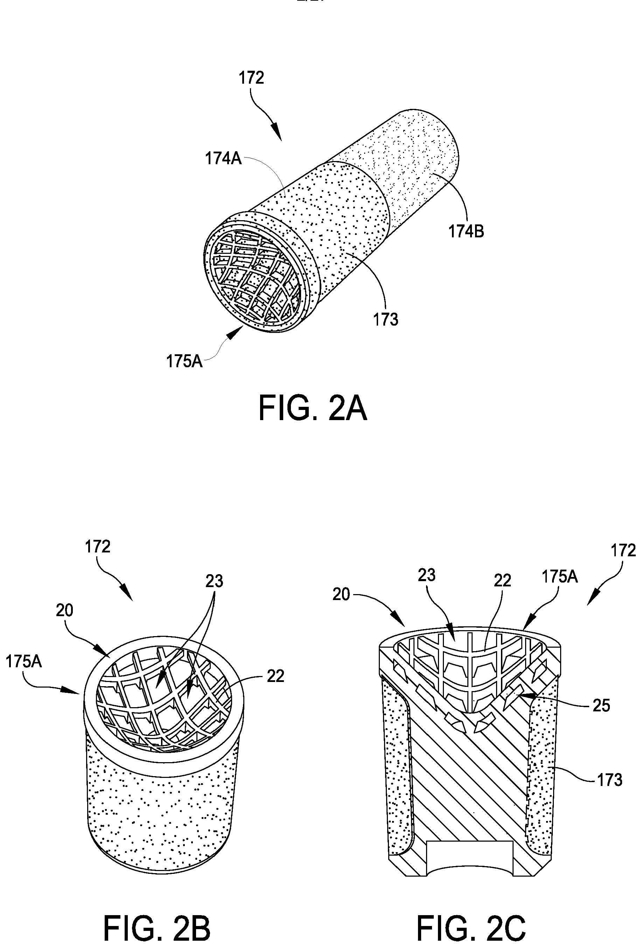

As shown in FIG. 2A, the porous metallic layer 173 can cover a first portion 174A of the outer surface of the distal portion 172 and a second (e.g., remaining) portion 174B of the distal portion 172 can include a solid metal.

Additionally, the porous metallic layer 173 can cover the full circumference of the distal portion 172 continuously or the porous metallic layer 173 can be provided in a discontinuous configuration covering just a portion or multiple portions of the exterior side wall surface of the distal portion 172. In the illustrated example shown in FIG. 1A, the porous metallic layer 173 is a continuous layer that covers the full circumference of the distal portion 172.

In some embodiments, the metallic distal portion 172 can be joined to the polymer base portion 171 by compression molding the polymer material directly to the metallic distal portion 172. Referring to FIG. 2A, to provide a structurally robust mechanical interface between the metallic distal portion 172 and the polymer base portion 172, a proximal end 175A of the metallic distal portion 172 (e.g., an end joining the polymer base portion 171) is configured in a manner that increases the surface area of the interface. Specifically, in the illustrated embodiment, the proximal end 175A is formed with a surface that has an interdigitated lattice structure 20.

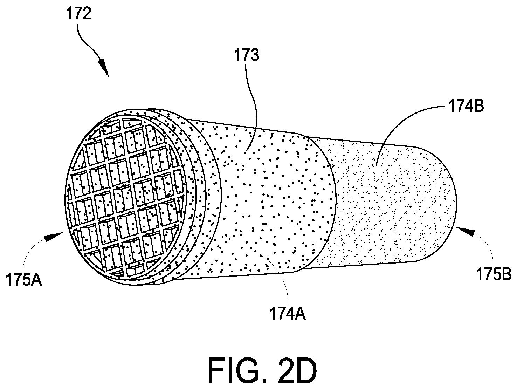

In some embodiments, the surface of the proximal end 175A of the metallic distal portion 172 has a conical shaped interdigitated lattice structure 20 where the top end of the conical shape points distally so that the proximal end 175A of the metallic distal portion 172 has a concave surface as shown in FIGS. 1B and 2A. The concave surface is concave toward the distal end 175B of the metallic distal portion 172. In some embodiments, the proximal end 175A of the metallic distal portion 172 can be flat or convex. An example of an embodiment where the proximal end 175A of the metallic distal portion 172 is flat is shown in FIG. 2D.

FIGS. 2B and 2C show the details of the interdigitated lattice structure 20. The interdigitated lattice structure comprises crisscrossing lattices 22 forming pockets 23 therebetween. The void spaces defined by the pockets 23 extend under the lattice work thus connecting the pockets 23 to their adjacent pockets. These connecting spaces 25 under the lattice work can be seen in the cross-sectional view shown in FIG. 2C. The interdigitated lattice structure 20 allows the polymer material to flow into the pockets and undercut spaces of the lattice structure during the compression molding process. When the polymer subsequently solidifies, the resulting interface comprises the interdigitated lattice structure into which the polymer material has infiltrated to form an interlocked structure comprising the polymer material and the metallic structure of the distal portion 172. The result is an interlocking interface forming a strong hybrid structure for the fixation post 170.

Many methods can be used to fabricate the metallic distal portion 172 that includes the interdigitated lattice structure. One method is by 3D printing with laser sintering. A more detailed discussion of an example of a manufacturing process is provided below.

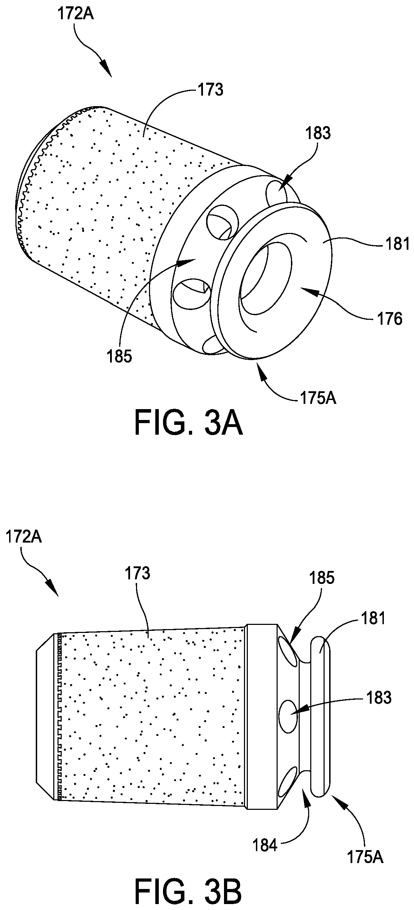

FIGS. 3A-3D are illustrations of a metallic distal portion 172A according to another embodiment. This second embodiment of the metallic distal portion 172A also has a solid metallic core whose side surface comprises a porous metallic layer 173 that promotes bone in-growth after implanted into a glenoid.

Same as for the metallic distal portion 172, the porous metallic layer 173 on the metallic distal portion 172A can cover the full circumference of the distal portion 172A continuously or the porous metallic layer 173 can be provided in a discontinuous configuration covering just a portion or multiple portions of the exterior side wall surface of the distal portion 172A. In the illustrated example shown in FIG. 3A, the porous metallic layer 173 is a continuous layer that covers the full circumference of the distal portion 172A.

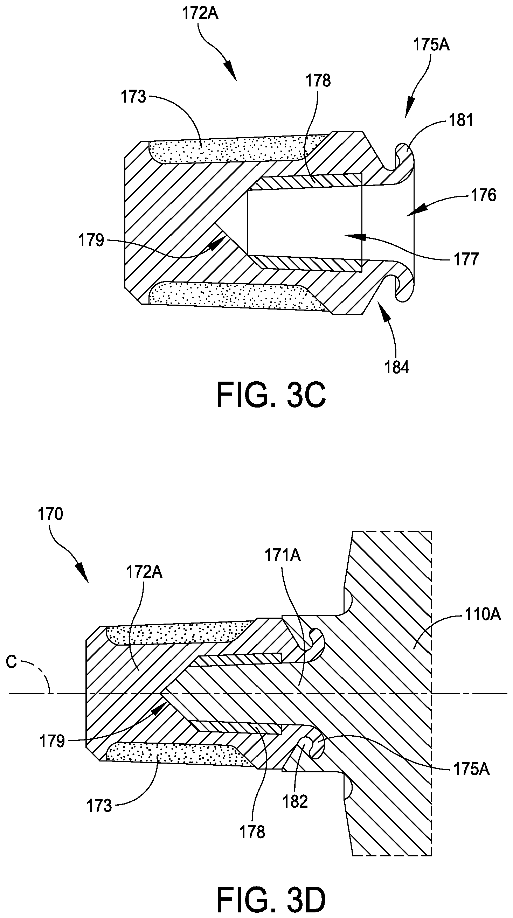

As shown in FIG. 3D, in this embodiment, the hybrid fixation post 170A is formed by the metallic distal portion 172A that is joined to a base portion 171A, where the base portion 171A is an extension of the polymer articular body 110A. In some embodiments, the metallic distal portion 172A can be joined to the polymer base portion 171A by compression molding the polymer material directly to the metallic distal portion 172A.

To provide a structurally robust mechanical interface between the metallic distal portion 172A and the polymer base portion 171A, the metallic distal portion 172A comprises at least three features that enhance the mechanical integrity of the interface between the metallic distal portion 172A and the polymer base portion 171. One such feature is that the proximal end 175A of the metallic distal portion 172A is provided with an opening 176 that extends distally into the body of the metallic distal portion 172A and defining a cavity 177. This cavity 177 allows the polymer material of the articular body 110A to flow into the cavity 177 during the compression molding process mentioned above and form the polymer base portion 171A of the fixation post 170A. The interior surface of the cavity 177 is provided with an interdigitated lattice structure portion 178 allowing the polymer material and the interior surface of the cavity 177 to form a very strong interlocking interface.

In some embodiments, the interdigitated lattice structure portion 178 can extend fully around the interior surface of the cavity 177 as a continuous surface. In some embodiments, the interdigitated lattice structure portion 178 can be provided as one or more discontinuous portions around the interior surface of the cavity 177. When provided as multiple portions, preferably, they should be positioned in radially symmetric arrangement.

The distal end of the cavity 177 is formed as a conical surface 179. The conical surface allows the polymer material to flow into the cavity and completely fill the cavity while minimizing voids during the compression molding process.

A second feature for enhancing the mechanical integrity of the interface between the metallic distal portion 172A and the polymer base portion 171A is the provision of a flared locking lip 181 at the proximal end 175A along the edge of the opening 176 as shown in FIGS. 3A-3D. As shown in the longitudinal cross-sectional views in FIGS. 3C-3D, the flared locking lip 181 is flared radially outward and turns partially toward the distal end 175B of the distal portion 172A, thus forming a hook-like cross-section. Thus, during the compression molding process, the polymer material of the articular body 110A flows around and under the flared locking lip 181 and forms an interlocking complementary lip portion 182 of the base portion 171A. The result is an interlocking interface that has an annular configuration providing radially symmetric engagement between the metallic distal portion 172A and the polymer base portion 171A.

A third feature for enhancing the mechanical integrity of the interface between the metallic distal portion 172A and the polymer base portion 171A is the provision of a plurality of flow ports 183 that provides fluid connection between the cavity 177 and the outside of the distal portion 172A in addition to the opening 176. The flow ports 183 provide additional flow paths for the polymer to flow into the cavity 177 during the compression molding process that forms the articular body 110A and simultaneously joins it to the distal portion 172A.

As can be seen in FIG. 3B, the distal portion 172A is configured with a narrow neck portion 184 near the flared locking lip 181. The narrow neck portion 184 has a sloped frusto-conical surface 185 that slopes radially inward toward the narrow neck portion 184. The plurality of the flow ports 183 are provided in the sloped frusto-conical surface 185 of the narrow neck portion 184 so that the flow ports 183 open toward the proximal end 175A of the metallic distal portion 172A, which is the direction from which the liquid polymer material would advance during a compression molding process. Thus, the flow ports 183 are oriented to readily receive the flow of polymer and provide multiple flow paths for the liquid polymer. This enables efficient flow of the liquid polymer into the cavity 177. Once the polymer solidifies forming the articular body 110A, the solid polymer material forming the base portion 171A extends into the cavity 177 via the opening 176 and also extends through each of the plurality of flow ports 183. This forms a plurality of polymer legs that interlock with the distal portion 172A providing additional connecting structures at the interface between the metallic distal portion 172A and the polymer base portion 171A.

The three features for enhancing the mechanical integrity of the interface between the metallic distal portion 172A and the polymer base portion 171A described above do not all have to be present in one hybrid fixation post 170A embodiment. Only one of the three features can be implemented in a hybrid fixation post 170A. In some embodiments, any partial combinations of the three features can be implemented in a hybrid fixation post 170A. For example, in some embodiments, the first and second features can be implemented in a hybrid fixation post 170A. In some embodiments, the first and third features can be implemented in a hybrid fixation post 170A. In some embodiments, the second and third features can be implemented in a hybrid fixation post 170A.

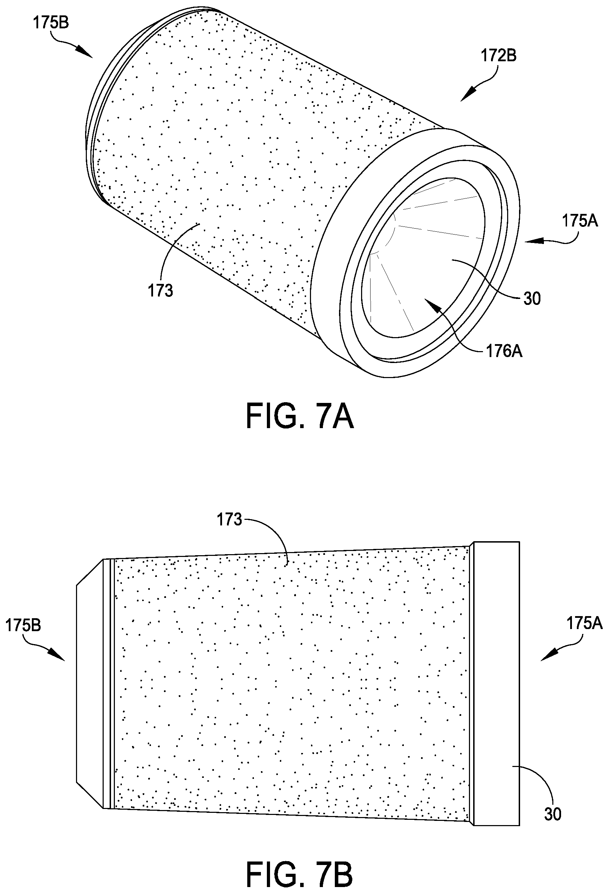

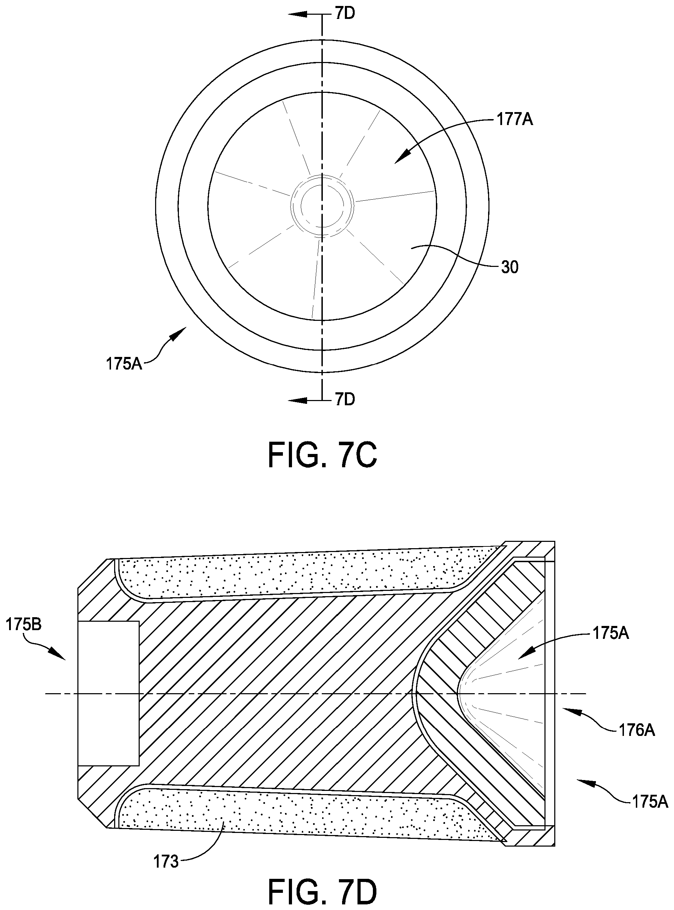

FIGS. 7A-7D illustrate a metallic distal portion 172B, in accordance with some embodiments of the present disclosure. The metallic distal portion 172B is similar to the metallic distal portions 172, 172A discussed above, and similar description is omitted herein.

In some embodiments, a proximal end 175A of the metallic distal portion 172B includes a conical shaped porous metal structure 30 where a top end of the conical shape points distally so that the proximal end 175A of the metallic distal portion 172B defines a concave opening 176A. The concave opening 176A is concave toward the distal end 175B of the metallic distal portion 172B. A cavity 177A defined by the conical shape of the porous metal structure 30 allows the polymer material of an articular body 110, 110A to flow into the cavity during 177A the compression molding process to form a base portion (e.g., base portion 171) of a fixation post (e.g., fixation post 170).

The porous metal allows a polymer material of an articular body 110, 110A to flow at least partially into the porous metal structure 30 during a compression molding process (or other molding process). When the polymer subsequently solidifies, the resulting interface includes the porous metal structure 30 into which the polymer material has infiltrated to form an interlocking structure. The porous metal structure 30 includes a porous metal material, such as a porous titanium or titanium-based alloy. The porous metal structure 30 may include the same porous metal material as a porous metallic layer 173 and/or may be different from the material of the porous metallic layer 173.

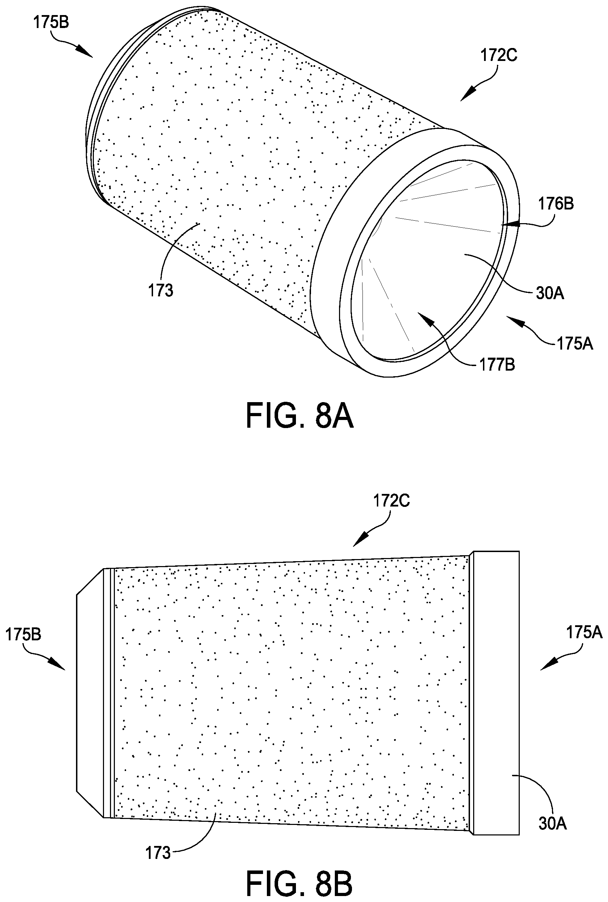

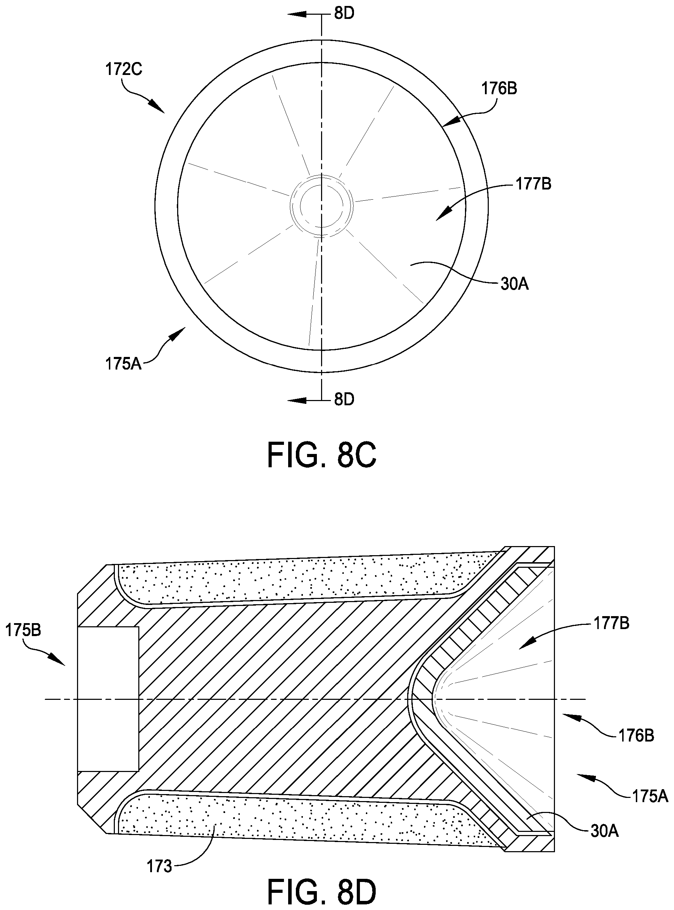

FIGS. 8A-8D illustrate a metallic distal portion 172C, in accordance with some embodiments of the present disclosure. The metallic distal portion 172C is similar to the metallic distal portion 172B discussed above, but has a thinner porous metal structure 30A as compared to the embodiment of FIGS. 7A-7C. For example, in some embodiments, the metallic distal portion 172B of FIGS. 7A-7C may have a porous metal structure 30 including a thickness of about 1 mm and the metallic distal portion 172C of FIGS. 8A-8D may have a porous metal structure 30A including a thickness of about 0.5 mm. Although specific embodiments are discussed herein, it will be appreciated that the thickness of a porous metal structure 30, 30A may be selected to be any suitable thickness to provide a desired interface thickness having one or more desired parameters, such as a pull-out strength.

As illustrated in FIGS. 8A-8C, in some embodiments, a thinner porous metal structure 30A may increase a depth of a cavity 177B formed by the porous metal structure 30A. For example, in the illustrated embodiment, the porous metal structure 30A has a distal end that is similarly positioned to a distal end of the porous metal structure 30 of FIGS. 7A-7C. However, because the porous metal structure 30A is thinner, the depth of the cavity 177B is greater than the depth of the cavity 177A. In other embodiments, the height of the porous metal structures 30, 30A may each be adjusted such that a depth of the cavity 177B is greater than, less than, or equal to the depth of cavity 177A.

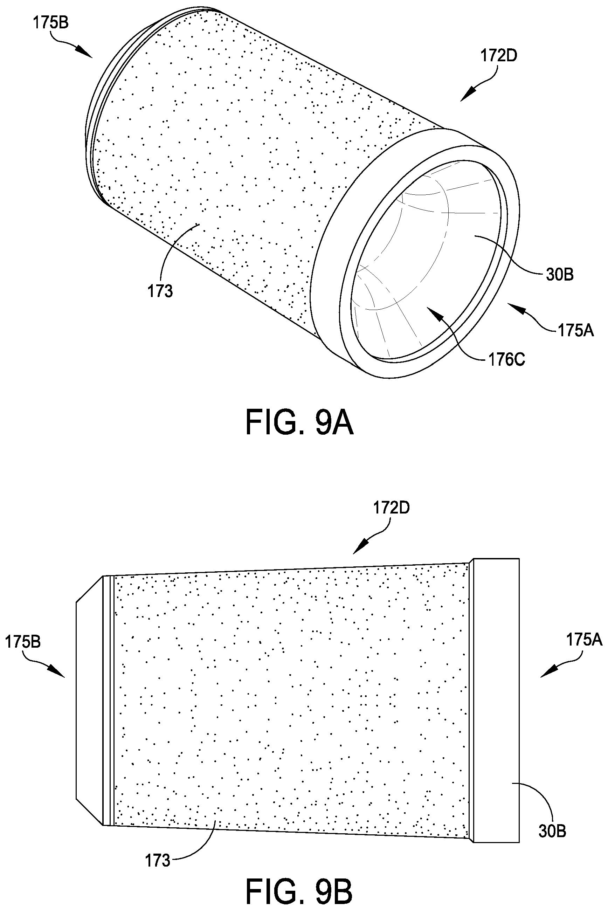

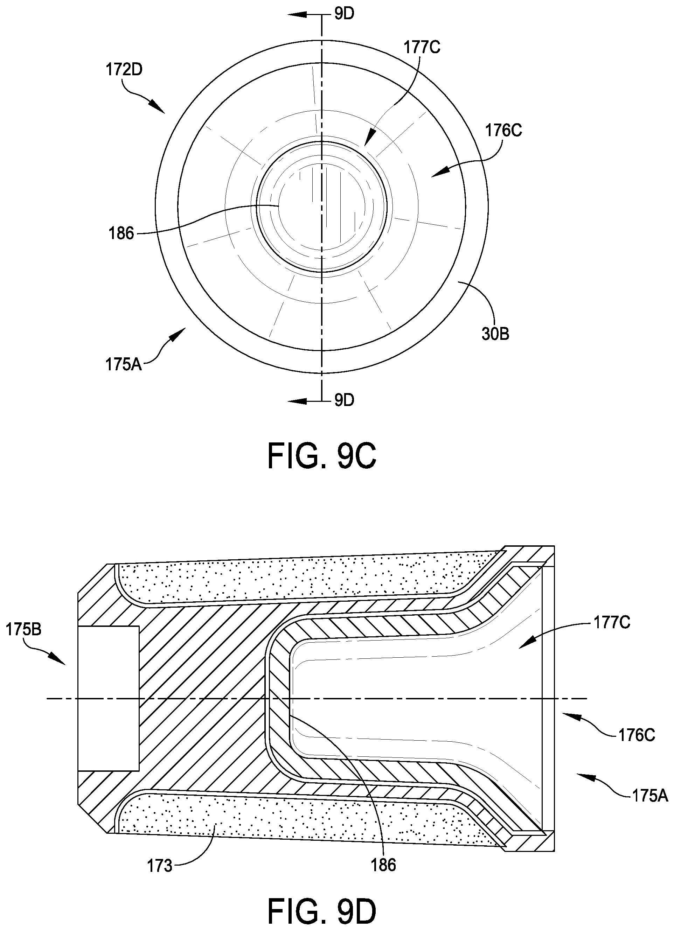

FIGS. 9A-9D illustrate a metallic distal portion 172D, in accordance with some embodiments of the present disclosure. The metallic distal portion 172D is similar to the metallic distal portions 172-172C discussed above, and similar description is omitted herein.

In some embodiments, a proximal end 175A of the metallic distal portion 172D includes a generically cylindrical shaped porous metal structure 30B having a flat internal bottom 186 such that the proximal end 175A of the metallic distal portion 172D defines a concave opening 176B and a cavity 177C. The concave opening 176B is concave toward the distal end 175B of the distal portion 172D. The cavity 177C defined by the porous metal structure 30B allows the polymer material of an articular body 110, 110A to flow into the cavity 177C during the compression molding process to form a base portion (e.g., base portion 171, 171A) of a fixation post (e.g., fixation post 170, 170A). In some embodiments, the concave opening 176B is defined by a flared or tapered proximal portion of the porous metal structure 30B.

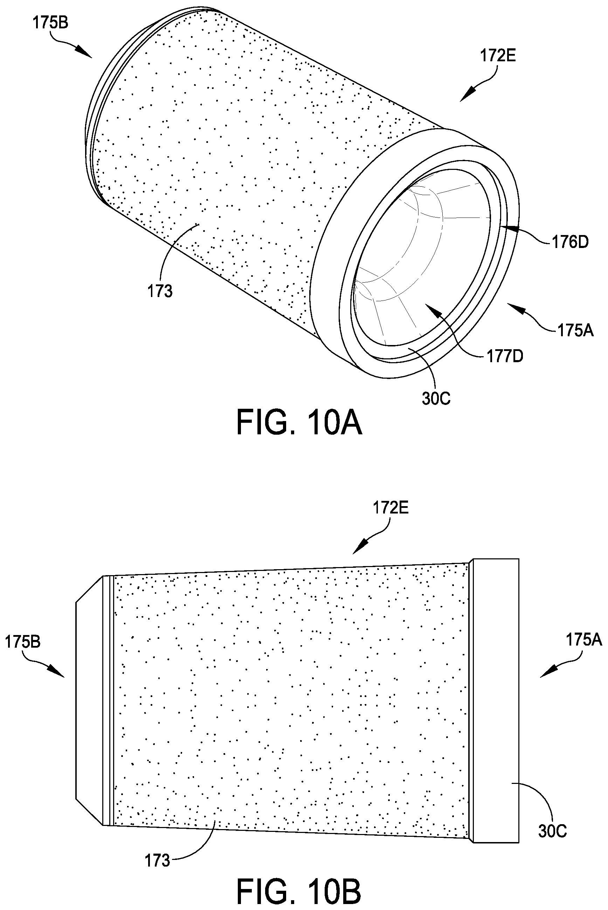

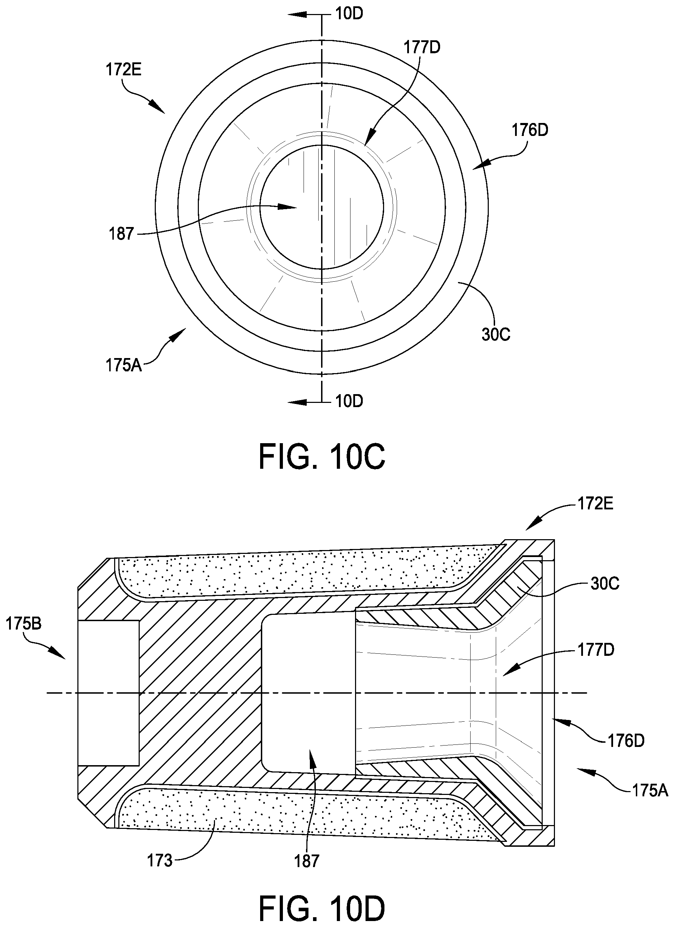

FIGS. 10A-10D illustrate a metallic distal portion 172E, in accordance with some embodiments of the present disclosure. The metallic distal portion 172E is similar to the metallic distal portion 172D discussed above, and similar description is omitted herein.

The metallic distal portion 172E includes a cavity 177D that is substantially similar to a corresponding portion of the metallic distal portion 172D. For example, the metallic distal portion 172E includes an opening 176C and a cavity 177D that are similar to the opening 176B and a proximal portion of the cavity 177C of the metallic distal portion 172D. The metallic distal portion 172E includes a region 187 having solid metal walls that corresponds to a distal portion of the cavity 177C. The region 187 provides a portion of the cavity 177D that does not include the porous metal structure 30C but still defines a void area for polymer to flow into during a molding process.

The region 187 may include the same material as a second portion 174B of the metallic distal portion 172E and/or may include a separate material. In some embodiments, the region 187 may include a non-porous version of a material similar to the material of the porous metal structure 30C, e.g., titanium or a titanium alloy.

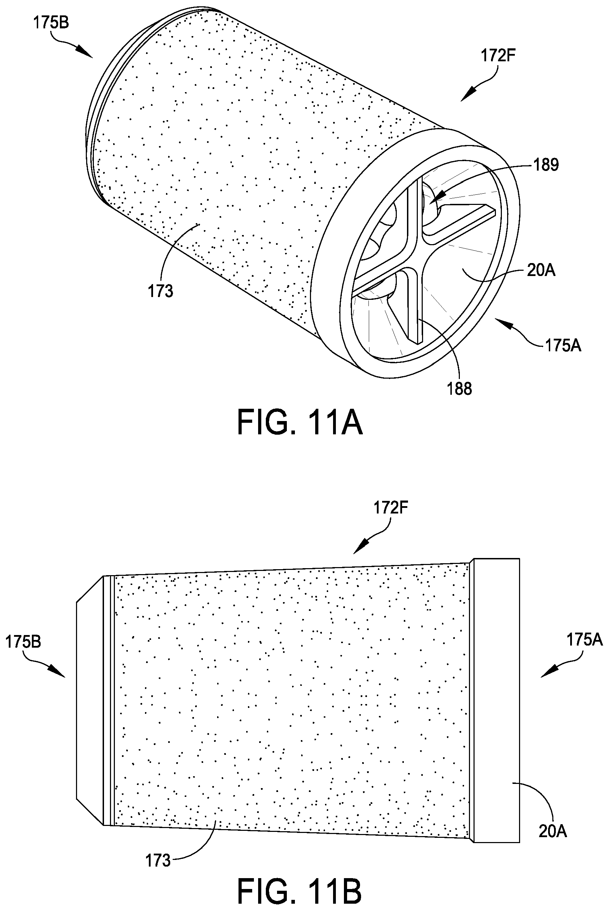

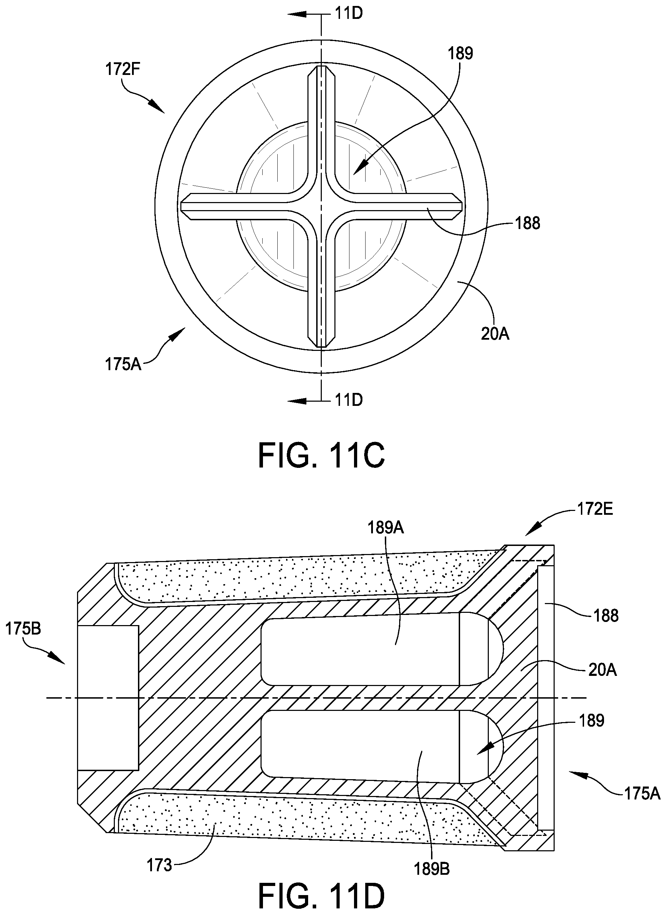

FIGS. 11A-11D illustrate a metallic distal portion 172F, in accordance with some embodiments of the present disclosure. The metallic distal portion 172F is similar to the metallic distal portions 172-172E discussed above, and similar description is omitted herein.

In some embodiments, a proximal end 175A of the metallic distal portion 172F includes a macro-cross structure 188 including a void portion 189 and an interdigitated lattice structure 20A. The void portion 189 may include solid metal walls 189A, 189B defining a void or cavity area for inflow of a polymer material. The interdigitated lattice structure 20A is similar to the interdigitated lattice structure 20 discussed above and allows a polymer material to flow into and bond with the interdigitated lattice structure 20 during a compression molding process.

In some embodiments, the interdigitated lattice structure 20A includes a different lattice structure as compared to the interdigitated lattice structure 20 of FIGS. 2A-2D. The interdigitated lattice structure 20A is defined at a proximal end 175A of the metallic distal portion 172F and is formed integrally with the portion 189.

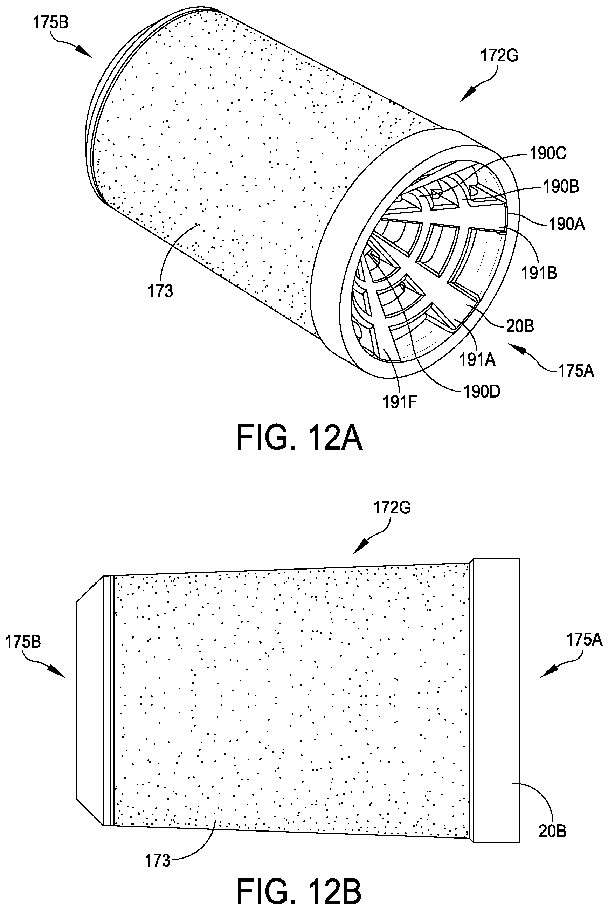

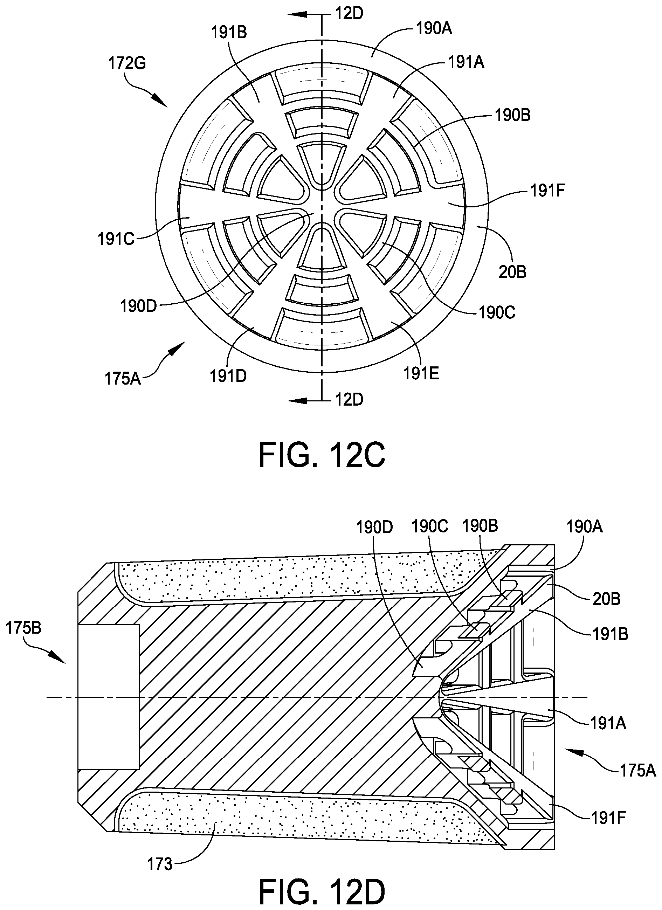

FIGS. 12A-12D illustrate a metallic distal portion 172G, in accordance with some embodiments of the present disclosure. The metallic distal portion 172G is similar to the metallic distal portions 172-172F discussed above, and similar description is not repeated herein.

In some embodiments, a proximal end 175A of the metallic distal portion 172G includes a radial mesh interdigitated lattice structure 20B. The radial mesh interdigitated lattice structure 20B includes concentric vertical layers 190A-190D having decreasing circumferences that are each coupled to vertical connection elements 191A-191D. Each of the vertical layers 190A-190D defines a set of concentric voids or openings. In some embodiments, a fourth concentric vertical layer 190D is at least partially embedded within a solid metal portion of the metallic distal portion 172G. Like the previously discussed interdigitated lattice structures 20, 20A, the radial mesh interdigitated lattice structure 20B allows a polymer material to flow between and bond with the radial mesh interdigitated lattice structure 20B during a compression molding process.

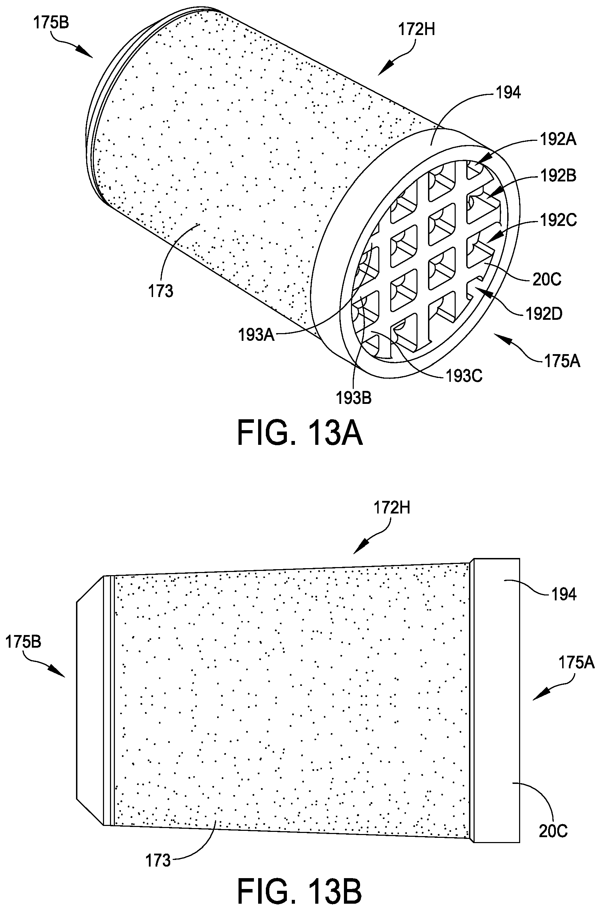

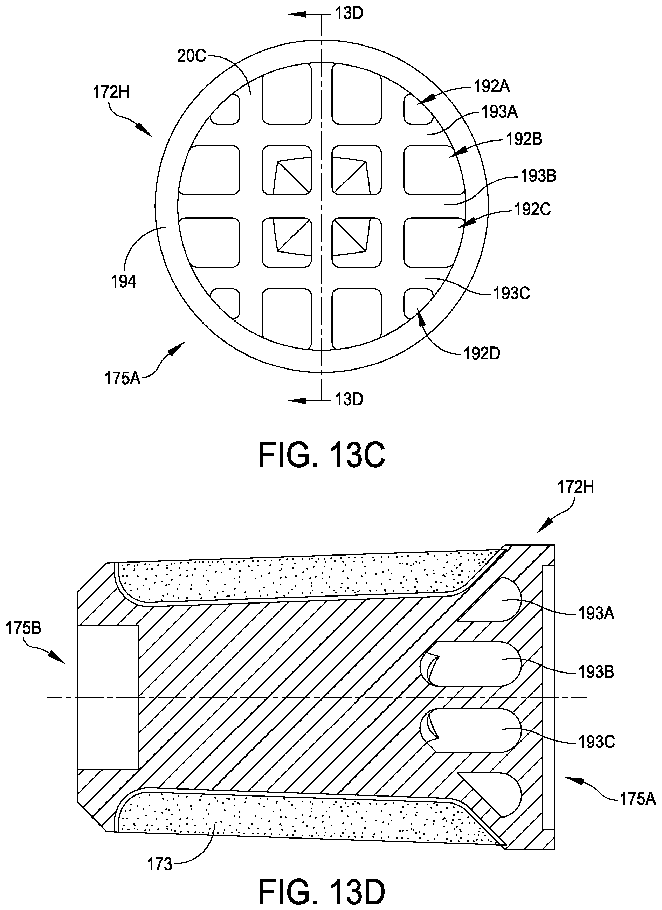

FIGS. 13A-13D illustrate a metallic distal portion 172H, in accordance with some embodiments of the present disclosure. The metallic distal portion 172H is similar to the metallic distal portions 172-172G discussed above, and similar description is not repeated herein.

In some embodiments, a proximal end 175A of the metallic distal portion 172H includes a coarse mesh interdigitated lattice structure 20C. The coarse mesh interdigitated lattice structure 20C includes coarse horizontal layers 192A-192D that are defined by horizontal members 193A-193C and an outer radial member 194. Like the previously discussed interdigitated lattice structures 20-20B, the radial mesh interdigitated lattice structure 20C allows a polymer material to flow between and bond with the radial mesh interdigitated lattice structure 20C during a compression molding process.

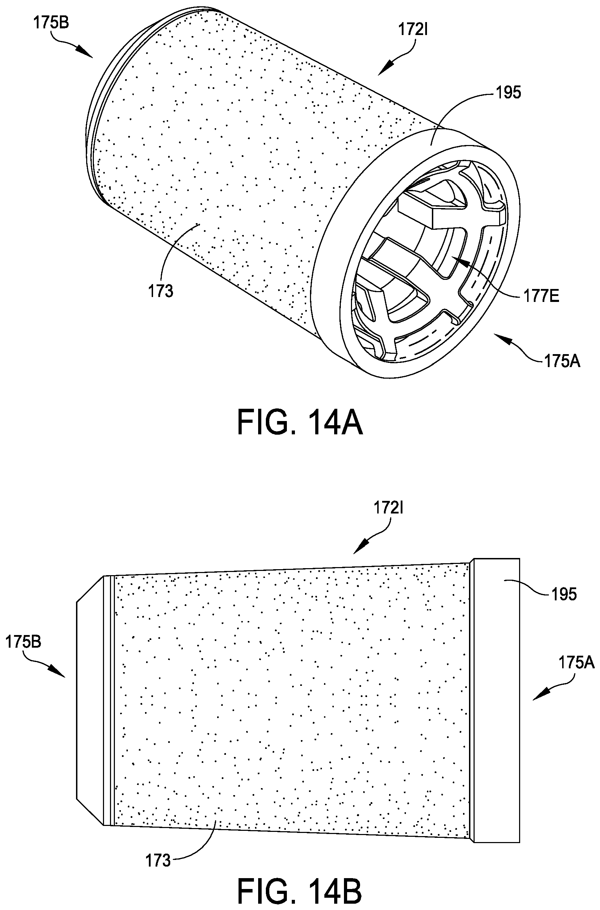

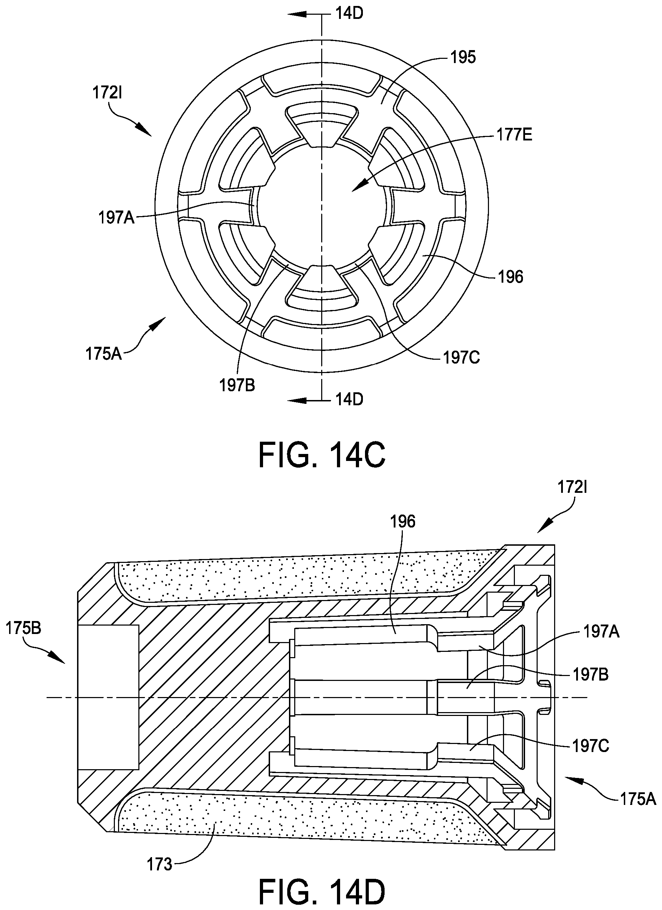

FIGS. 14A-14D illustrates a cross-sectional view of a metallic distal portion 172I, in accordance with some embodiments of the present disclosure. The metallic distal portion 172I is similar to the metallic distal portions 172-172G discussed above, and similar description is not repeated herein.

In some embodiments, the metallic distal portion 172I includes a transformer component 195. The transformer component 195 defines an opening 177E that has a depth equal to or greater than half the overall length of the metallic distal portion 172I. The interior surface 196 of the transformer component 195 may include one or more features 197A-197C that provide joint or tension points for a polymer material that is flowed into the opening 177E during a compression molding process.

It will be appreciated that features of each of the disclosed hybrid fixation posts 170, 170A and/or metallic distal portions 172-172I may be utilized in any combination. In general, each of the disclosed metallic distal portions 172-172I are configured to decrease slip-out (e.g., disconnection of the hybrid fixation post 170, 170A) and toggle movement and/or increase pull-out strength. For example, each of the disclosed metallic distal portions 172-172I include one or more features that allow a polymer material of a base portion 171, 171A to couple to and/or connect to the metallic distal portions 172-172I.

In some embodiments, a pull-out strength for each of the disclosed metallic distal portions 172-172I may be equivalent, with variations in the designs providing different fatigue resistances and/or toggle allowances. For example, in some embodiments including larger voids, a higher quantity of bulk polymer material may improve fatigue resistance. As another example, in some embodiments, one or more features of the metallic distal portions 172-172I may prevent cutting or wearing of the polymer material by the metallic elements of the metallic distal portions (e.g., utilizing specific an interdigitated lattice structures to prevent cutting) and/or to compensate for expected cutting (e.g., by increasing a void space for polymer inflow).

In various embodiments, one or more of an interdigitated lattice structure, a porous material structure, a cavity (or void space), a macro feature (e.g., an anchoring metal section), and/or any other disclosed elements may be combined to achieve desired parameters of a hybrid fixation post. In some embodiments, combinations of one or more of these features maintain connections between the polymer material of the base portion and the metal material of the distal portion during compression molding and/or cooling of the polymer material.

With reference again to FIG. 1A, in some embodiments, the anatomic glenoid implant 100, further comprises one or more additional fixation features 140 extending from the medial surface 113 that are arranged to prevent rotation of the articular body 110 with respect to the glenoid when the glenoid implant 100 is implanted in the glenoid. Each of the one or more fixation features 140 can be a structure in the form of a post, as in the illustrated example of FIG. 1A. However, the particular structural shape of the fixation features 140 is not limited to that example and can be configured in any shape that would allow fixation to the glenoid. The glenoid would be prepared appropriately to receive the one or more of the fixation features 140 depending on the shape of the fixation features 140.

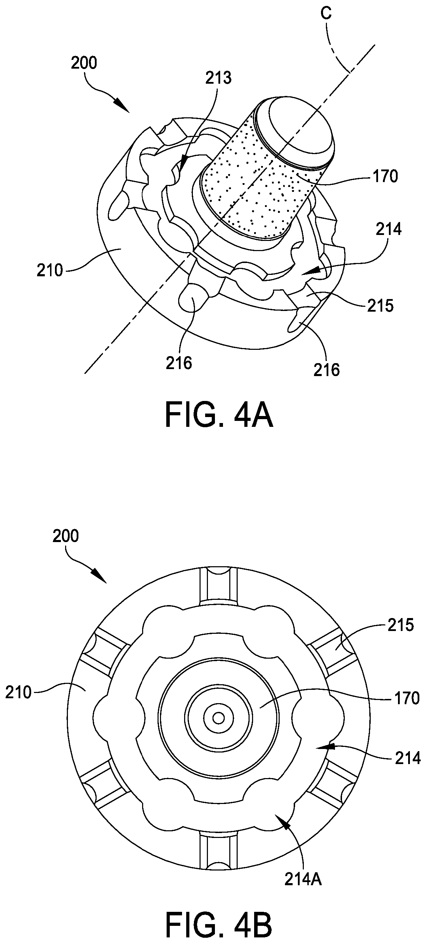

FIGS. 4A-4C are illustrations showing another embodiment of an anatomic glenoid implant 200 having a hybrid fixation post 170. The glenoid implant 200 comprises an articular body 210, and at least one hybrid fixation post 170. The articular body 210 comprises an articulation surface 212 and a medial surface 213 on the opposite side of the articular body.

The hybrid fixation post 170 has the same structure and composition as the various embodiments described herein.

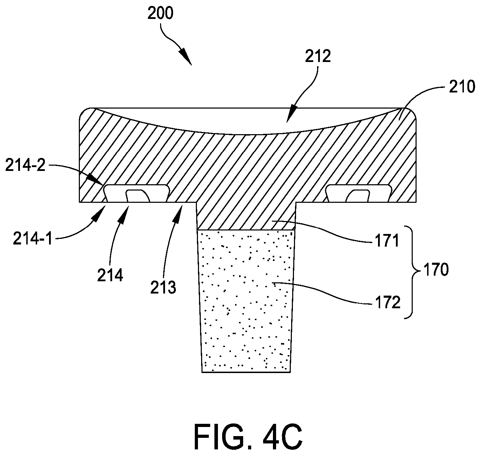

The articular body 210 comprises a cement channel 214 provided on the medial surface 213 for holding an amount of bone cement when the glenoid implant 200 is being implanted in a glenoid. The cement channel 214 extends circumferentially around and concentric with the central axis C of the glenoid implant 200. As shown in the cross-sectional view in FIG. 4C, the cement channel 214 has a dovetail cross-section such that the channel's opening 214-1 has a width narrower than the channel's base 214-2. This undercut shape of the cement channel aids in holding the bone cement and enhance adhesion to the bone.

The cement channel 214 can also comprise a plurality of anti-rotation features for the bone cement that are in the form of widened pocket sections 214A along the length of the cement channel 214. The pocket sections 214A hold additional amount of bone cement and help prevent rotation of the glenoid implant 200.

The articular body 210 can also be provided with a plurality of radially extending ancillary cement channels 215 that connect the main cement channel 214 to exterior side of the articular body 210. These ancillary cement channels 215 extend along the medial surface 213 and allow excess bone cement in the main cement channel 214 to escape as the glenoid implant 200 is being positioned into the prepared glenoid surface.

The depth of the cement channel 214, the ancillary cement channels 215, and the widened pocket sections 214A can be in the range of 1 - 1.5 mm. In some embodiments, the widened pocket sections 214A can be deeper than the channel 214 and the ancillary channels 215.

Furthermore, in some embodiments, the ancillary cement channels 215 can be extended further along the side wall 210-S of the articular body 210 at least partially toward the articular surface forming an extended portion 216 of the ancillary cement channel. This extended portion 216 allows additional room for excess bone cement to escape as the glenoid implant 200 is being positioned into the prepared glenoid surface.

The illustrated example of the articular body 210 has a circular or generally cylindrical shape when configured for inlaid applications where the glenoid implant is inset into a prepared glenoid. However, the articular body 210 can be formed to have different shapes to accommodate different conditions of the receiving glenoid. In those embodiments, the pattern of the channel 214 For those embodiments, the procedure for preparing the glenoid surface would be modified accordingly.

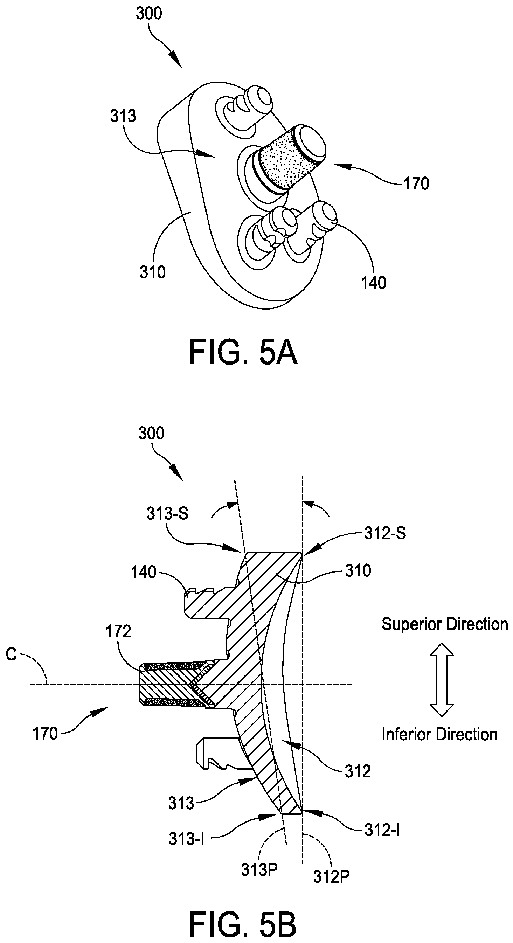

FIGS. 5A-5E are illustrations showing additional embodiments of an anatomic glenoid implant having a hybrid fixation post that are also configured to provide augmentation in the superior direction of the patient's glenoid. The anatomic glenoid implant can be used in an on-lay or inlaid application on a glenoid. Referring to FIGS. 5A-5B, the glenoid implant 300 comprises an articular body 310 that comprises an articulation surface 312, and a medial surface 313 opposite from the articular surface. A hybrid fixation post 170 according to one of the disclosed embodiments can be provided and extend from the medial surface 313.

FIG. 5B is a cross-sectional view of the glenoid implant 300 where the section is taken in superior-inferior direction and through the center of the hybrid fixation post 170. As shown, although the articular body 310 is a curved form, the overall shape of the articular body 310 will be described as being wedge-shaped with the superior side of the articular body 310 being thicker than the inferior end to augment the superior portion of the patient's glenoid. The articular body can be configured to provide any desired degree of angulation to provide augmentation in the superior direction. An example is an angulation of 7.5 degrees of superior augmentation shown in FIG. 5B. This angle will be referred to herein as "a superior augmentation angle." The superior augmentation angle is defined as the angle between a lateral side plane 312P and a medial side plane 313P.

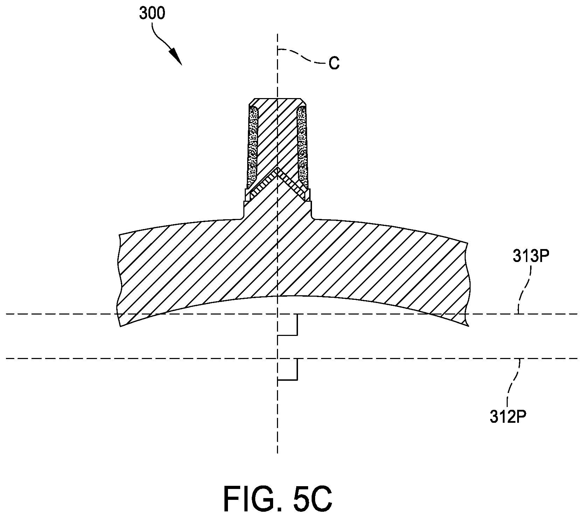

The lateral side plane 312P is defined as a plane that intersects the most-superior point 312-S of the articulation surface 312 and the most-inferior point 312-I of the articulation surface 312 and is also orthogonal to the longitudinal axis C of the hybrid fixation post 170 when viewed from superior to inferior direction, which is shown in FIG. 5C. The medial side plane 313P is defined as a plane that intersects the most-superior point 313-S of the medial surface 313 and the most-inferior point 313-I of the medial surface 313 and is also orthogonal to the longitudinal axis C of the hybrid fixation post 170 when viewed from superior to inferior direction which is shown in FIG. 5C.

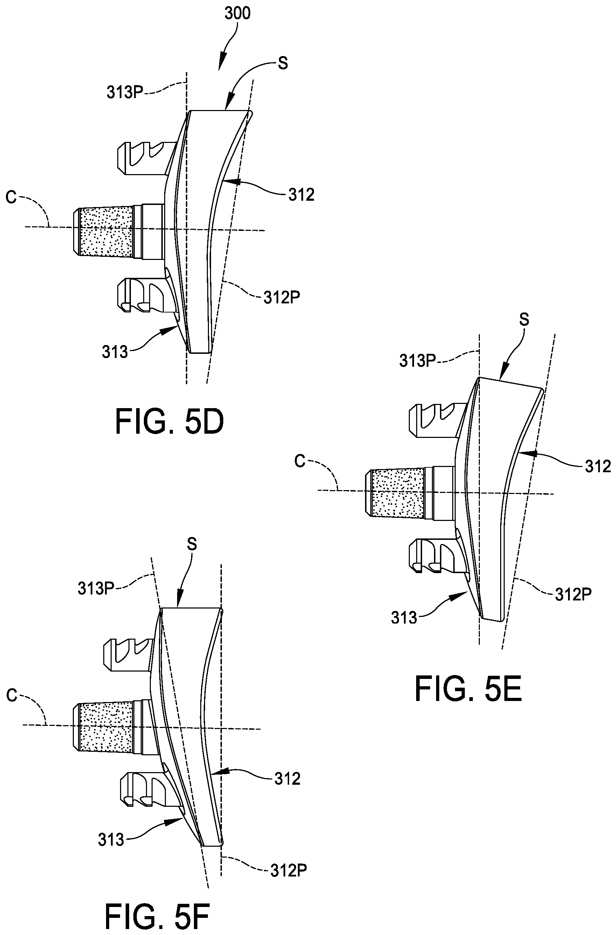

FIG. 5D shows an embodiment of the augmented glenoid implant 300 where the hybrid fixation post 170 is oriented orthogonal to the medial side plane 313P. The longitudinal axis C of the hybrid fixation post 170 is orthogonal to the medial side plane 313P. Additionally, the sidewall S along the perimeter of the articular body 310 is parallel to the hybrid fixation post 170.

FIG. 5E shows an embodiment of the augmented glenoid implant 300 where the hybrid fixation post 170 is oriented orthogonal to the medial side plane 313P. The longitudinal axis C of the hybrid fixation post 170 is orthogonal to the medial side plane 313P. However, in this embodiment, the sidewall S along the perimeter of the articular body 310 is not parallel to the hybrid fixation post 170.

FIG. 5F shows an embodiment of the augmented glenoid implant 300 where the hybrid fixation post 170 is oriented orthogonal to the lateral side plane 312P rather than the medial side plane 313P. The longitudinal axis C of the hybrid fixation post 170 is orthogonal to the lateral side plane 312P. In this embodiment, the sidewall S along the perimeter of the articular body 310 is parallel to the hybrid fixation post 170.

### [Manufacturing process]

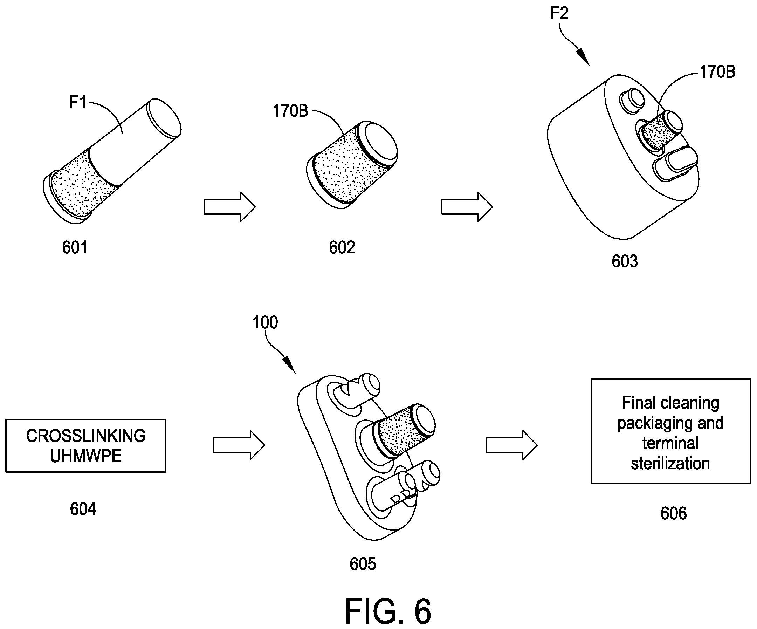

FIG. 6 shows the general process flow for fabricating the glenoid implant 100 that comprises a hybrid fixation post 170. At step 601, the initial metal post form F1 can be fabricated using a 3D printing method that includes laser sintering and heat treatment. Next, at step 602, the initial metal post form F1 is machined to the finished metallic distal portion 170B that meets the final dimensional specification. At step 603, an initial articular body form F2 is formed on to the metallic distal portion 170B by compression molding UHMWPE, directly onto the finished metallic distal portion 170B. At step 604, the initial articular body form F2 is exposed to a curing cycle allowing the UHMWPE to crosslink and polymerize. At step 605, the cured initial articular body form F2 is machined into the final glenoid implant dimensions. At step 606, the finished glenoid implant is cleaned and packaged.

Also within the scope of the present disclosure is a surgical kit comprising one or more of the disclosed glenoid implants. Each of the one or more glenoid implants in the kit can be any one of the embodiments disclosed herein.

Unless otherwise expressly stated, it is in no way intended that any method set forth herein be construed as requiring that its steps be performed in a specific order, nor that with any apparatus, specific orientations be required, unless specified as such. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred, in any respect. This holds for any possible non-express basis for interpretation, including: matters of logic with respect to arrangement of steps, operational flow, order of components, or orientation of components; plain meaning derived from grammatical organization or punctuation, and; the number or type of embodiments described in the specification.

Various embodiments of glenoid implants are disclosed herein.

In a first embodiment, a glenoid implant includes an articular body formed of a polymer material and comprising: an articulation surface and a medial surface on an opposite side of the articular body from the articulation surface and a hybrid fixation post extending distally from the medial surface of the articular body. The hybrid fixation post includes a hybrid composition that includes a base portion that is an extension of the articular body and a distal portion including a metallic structure. The base portion and the distal portion are joined in a manner in which an interface is formed between the base portion and a proximal end of the distal portion. The interface includes an interlocked structure comprising the polymer material and the metallic structure.

In a second embodiments, the glenoid implant of the first embodiment wherein the metallic structure comprises an interdigitated lattice structure, a porous metal material, a material defining a void space, a macro metal feature that provides one or more contact points, or any combination thereof.

In a third embodiment, the glenoid implant of either of the preceding embodiments wherein the metallic structure is provided at the proximal end of the distal portion.

In a fourth embodiment, the glenoid implant of any of the preceding embodiments wherein the metallic structure has a conical shape that is concave toward a distal end of the hybrid fixation post.

In a fifth embodiment, the glenoid implant of any of the preceding embodiments wherein the polymer material is an ultra-high-molecular-weight polyethylene.

In a sixth embodiment, the glenoid implant of any of the preceding embodiments, wherein the distal portion includes a solid metallic core having an exterior side wall surface including a porous metallic layer.

In a seventh embodiment, the glenoid implant of the sixth embodiment wherein the porous metallic layer covers a full circumference of the exterior side wall surface of the distal portion.

In an eighth embodiment, the glenoid implant of the sixth embodiment wherein the porous metallic layer covers the exterior side wall surface of the distal portion continuously.

In a ninth embodiment, the glenoid implant of the sixth embodiment wherein the porous metallic layer covers the exterior side wall surface of the distal portion in a discontinuous configuration including one or more portions.

In a tenth embodiment, the glenoid implant of any of the preceding embodiments including one or more additional fixation features extending from the medial surface of the articulation body that are arranged to prevent rotation of the articular body with respect to the glenoid when the glenoid implant is implanted in the glenoid.

In an eleventh embodiment, the glenoid implant of the tenth embodiment wherein each of the one or more fixation features comprises a post.

In a twelfth embodiment, a glenoid implant includes an articular body formed of a polymer material including an articulation surface and a medial surface on an opposite side of the articular body from the articulation surface. The glenoid implant further includes a hybrid fixation post extending distally from the medial surface including a base portion that is an extension of the articular body and a distal portion including a metallic structure. The base portion and the distal portion are joined in a manner in which an interface is formed between the base portion and a proximal end of the distal portion. The interface includes an interlocked structure comprising the polymer material and the metallic structure of the distal portion. The proximal end of the distal portion defines an opening that extends distally into the distal portion and defines a cavity that is filled with the polymer material. An interior surface of the cavity is provided with the metallic structure.

In a thirteenth embodiment, the glenoid implant of the twelfth embodiment wherein the metallic structure includes an interdigitated lattice structure, a porous metal material, a macro metal feature that provides one or more contact points, or any combination thereof.

In a fourteenth embodiment, the glenoid implant of the twelfth or thirteenth embodiment wherein the metallic structure extends fully around the interior surface of the cavity as a continuous surface.

In a fifteenth embodiment, the glenoid implant of the any of the twelfth through fourteenth embodiments, wherein the metallic structure is provided as one or more discontinuous portions around the interior surface of the cavity.

In a sixteenth embodiment, the glenoid implant of the any of the twelfth through fifteenth embodiments wherein the distal portion comprises a flared locking lip at the proximal end along an edge of the opening, wherein the flared locking lip is flared radially outward and turns partially toward the distal end of the distal portion.

In a seventeenth embodiment, the glenoid implant of the any of the twelfth through sixteenth embodiments wherein the distal portion comprises a plurality of flow ports that provide fluid connection between the cavity and an exterior of the distal portion.

In an eighteenth embodiment, the glenoid implant of the seventeenth embodiment wherein the distal portion comprises a neck portion including a sloped frusto-conical surface that slopes radially inward, wherein each flow port of the plurality of flow ports are provided in the sloped frusto-conical surface.

In an nineteenth embodiment, the glenoid implant of the any of the twelfth through eighteenth embodiments wherein the articular body is formed of ultra-high-molecular-weight polyethylene.

In a twentieth embodiment, the glenoid implant of the any of the twelfth through nineteenth embodiments wherein the distal portion comprises an exterior side wall surface that includes a porous metallic layer.

In a twenty-first embodiment, the glenoid implant of the twentieth embodiment wherein the porous metallic layer covers a full circumference of the exterior side wall of the distal portion.

In a twenty-second embodiment, the glenoid implant of the twenty-first embodiment wherein the porous metallic layer covers the exterior side wall of the distal portion continuously.

In a twenty-third embodiment, the glenoid implant of the twenty-first embodiment wherein the porous metallic layer covers the exterior side wall of the distal portion in a discontinuous configuration including one or more portions.

In a twenty-fourth embodiment, the glenoid implant of any of the twelfth through twenty-third embodiments including one or more additional fixation features extending from the medial surface of the articular body that are arranged to prevent rotation of the articular body with respect to the glenoid when the glenoid implant is implanted in the glenoid.

In a twenty-fifth embodiment, the glenoid implant of any of the twelfth through twenty-fourth embodiments wherein each of the one or more fixation features is in the form of a post.

In a twenty-sixth embodiment, the glenoid implant of any of the preceding embodiments wherein a central axis of the articular body is defined through the hybrid fixation post and further including a channel provided on the medial surface and extending circumferentially around and concentric with the central axis of the articular body.

In a twenty-seventh embodiment, the glenoid implant of the twenty-sixth embodiment wherein the channel has a dovetail cross-section such that the channel's opening has a width narrower than the channel's base.

In a twenty-eighth embodiment, the glenoid implant of either of the twenty-sixth embodiment or the twenty-seventh embodiment wherein the channel comprises a plurality of anti-rotation features that are in the form of widened pocket sections along the length of the channel.

In a twenty-ninth embodiment, the glenoid implant of any of the twenty-sixth embodiment through the twenty-eighth embodiment including a plurality of radially extending ancillary channels that connect the channel to exterior side of the articular body.

In a thirtieth embodiment, the glenoid implant of the twenty-ninth embodiment wherein the ancillary channels are extended further along side wall of the articular body at least partially toward the articular surface thus forming an extended portion of the ancillary channel.

In a thirty-first embodiment, a method of manufacturing any of the preceding glenoid implants.

In a thirty-second embodiment, a surgical kit including a glenoid implant of any of first through thirtieth embodiments.

In a thirty-third embodiment, a method of forming a glenoid implant includes forming distal portion of a hybrid fixation post including a metallic structure and forming an articular body including an articulation surface, a medial surface on an opposite side of the articular body from the articulation surface, and a base portion of the hybrid fixation post extending distally from the medial surface. The articular body includes a polymer material. The base portion and the distal portion of the hybrid fixation post are joined in a manner in which an interface is formed between the base portion and the distal portion. The interface includes an interlocked structure comprising the polymer material and the metallic structure.

In a thirty-fourth embodiment, the method of forming a glenoid implant of the thirty-first embodiment or the thirty-third embodiment, wherein the distal portion of the hybrid fixation post is formed using additive manufacturing.

In a thirty-fifth embodiment, the method of forming a glenoid implant of the thirty-first embodiment or the thirty-third embodiment, wherein the distal portion of the hybrid fixation post is formed using subtractive manufacturing.

In a thirty-sixth embodiment, the method of forming a glenoid implant of the thirty-first embodiment or the thirty-third embodiment, wherein the distal portion of the hybrid fixation post is formed using both additive and subtractive manufacturing.

## Claims

1. A glenoid implant, comprising:
an articular body formed of a polymer material and comprising: an articulation surface and a medial surface on an opposite side of the articular body from the articulation surface; and
a hybrid fixation post extending distally from the medial surface of the articular body, wherein the hybrid fixation post comprises a hybrid composition that includes:
a base portion that is an extension of the articular body; and
a distal portion comprising a metallic structure, wherein the base portion and the distal portion are joined in a manner in which an interface is formed between the base portion and a proximal end of the distal portion,
wherein the interface comprises an interlocked structure comprising the polymer material and the metallic structure.

2. The glenoid implant of claim 1, wherein the metallic structure comprises an interdigitated lattice structure, a porous metal material, a material defining a void space, a macro metal feature that provides one or more contact points, or any combination thereof.

3. The glenoid implant of claim 2, wherein the metallic structure is provided at the proximal end of the distal portion.

4. The glenoid implant of claim 1, wherein the metallic structure has a conical shape that is concave toward a distal end of the hybrid fixation post.

5. The glenoid implant of claim 1, wherein the polymer material comprises an ultra-high-molecular-weight polyethylene.

6. The glenoid implant of claim 1, wherein the distal portion comprises a solid metallic core having an exterior side wall surface comprising a porous metallic layer.

7. The glenoid implant of claim 6, wherein the porous metallic layer covers a full circumference of the exterior side wall surface of the distal portion.

8. The glenoid implant of claim 6, wherein the porous metallic layer covers the exterior side wall surface of the distal portion continuously.

9. The glenoid implant of claim 6, wherein the porous metallic layer covers the exterior side wall surface of the distal portion in a discontinuous configuration including one or more portions.

10. The glenoid implant of claim 1, further comprising one or more additional fixation features extending from the medial surface of the articulation body that are arranged to prevent rotation of the articular body with respect to the glenoid when the glenoid implant is implanted in the glenoid.

11. The glenoid implant of claim 10, wherein each of the one or more fixation features comprises a post.

12. The glenoid implant of any of the preceding claims, wherein the proximal end of the distal portion defines an opening that extends distally into the distal portion and defines a cavity that is filled with the polymer material, and wherein an interior surface of the cavity is provided with the metallic structure.

13. The glenoid implant of claim 12, wherein the metallic structure extends fully around the interior surface of the cavity as a continuous surface.

14. The glenoid implant of either of claims 12 or 13, wherein the distal portion comprises a plurality of flow ports that provide fluid connection between the cavity and an exterior of the distal portion.

15. A method of forming a glenoid implant of any of the preceding claims.
